# EUROPÄISCHE PATENTSCHRIFT

(11) **EP 4 417 144 B1**
(45) Veröffentlichungstag und Bekanntmachung des Hinweises auf die Patenterteilung: **14.01.2026**
(21) Anmeldenummer: 24157261.9
(22) Anmeldetag: 13.02.2024
(51) Int. Cl.: A61B 17/16

(54) **CHIRURGISCHES INSTRUMENT**
SURGICAL INSTRUMENT
INSTRUMENT CHIRURGICAL

(30) Priorität: 14.02.2023 DE 102023103519
(43) Veröffentlichungstag der Anmeldung: 21.08.2024
(73) Patentinhaber: BAK Kohler Medical KG, 78579 Neuhausen ob Eck (DE)
(72) Erfinder: Stump, Klaus, 78579 Neuhausen ob Eck (DE); Kohler, Andreas, 78253 Eigeltingen/Honstetten (DE)
(74) Vertreter: Baldus, Oliver

(56) Entgegenhaltungen:
- WO-A1-2021/259066
- DE-A1- 102010 006 846
- DE-B4- 102006 012 754
- DE-B4- 19 921 614
- US-A1- 2015 201 950

## Beschreibung

Die vorliegende Erfindung betrifft ein chirurgisches Instrument zum Stanzen von Knochen.

Die Druckschrift EP 2 213 254 B1 betrifft ein chirurgisches Instrument mit einem Schaft und mit einem relativ zum Schaft längsverschiebbaren Schiebeteil. Derartige Instrumente sind bei der Reinigung unhandlich.

Es ist die technische Aufgabe der vorliegenden Erfindung, ein chirurgisches Instrument bereitzustellen, das auf einfachere Weise gereinigt werden kann.

Diese technische Aufgabe wird durch Gegenstände nach der unabhängigen Anspruch gelöst. Technisch vorteilhafte Ausführungsformen sind Gegenstand der abhängigen Ansprüche, der Beschreibung und der Zeichnungen.

Gemäß der Erfindung wird die technische Aufgabe durch ein chirurgisches Instrument zum Stanzen von Knochen nach Anspruch 1 gelöst, mit einem Schaft mit einem ersten Schneidelement; einem Schiebeteil mit einem zweiten Schneidelement, das in einer Arbeitsstellung verschiebbar an dem Schaft angeordnet ist; und einer Klappvorrichtung zum schnabelartigen Aufklappen des Schiebeteils und des Schafts in einer Reinigungsstellung, die eine Spreizvorrichtung zum Spreizen des Schiebeteils und des Schafts umfasst. In der Reinigungsstellung ist das Schiebelement gesichert, so dass es nicht versehentlich verriegelt werden kann, wenn der Schieber nach oben geklappt ist.

In einer technisch vorteilhaften Ausführungsform des chirurgischen Instruments umfasst die Spreizvorrichtung eine Spreizfeder. Dadurch wird beispielsweise der technische Vorteil erreicht, dass die Spreizvorrichtung mit geringem Aufwand realisiert werden kann.

In einer weiteren technisch vorteilhaften Ausführungsform des chirurgischen Instruments ist die Spreizvorrichtung zwischen dem Schaft und dem Schiebeteil angeordnet. Dadurch wird beispielsweise der technische Vorteil erreicht, dass ein kompakter Aufbau erzielt wird.

In einer weiteren technisch vorteilhaften Ausführungsform des chirurgischen Instruments ist die Spreizvorrichtung ausgebildet, das Schiebeteil gegen ein Anschlagselement zu drücken. Dadurch wird beispielsweise der technische Vorteil erreicht, dass der maximale Öffnungswinkel in der Reinigungsstellung begrenzt wird.

In einer weiteren technisch vorteilhaften Ausführungsform des chirurgischen Instruments ist das Anschlagselement fest positioniert. Dadurch wird beispielsweise der technische Vorteil erreicht, dass der maximale Öffnungswinkel in der Reinigungsstellung unveränderbar ist.

In einer weiteren technisch vorteilhaften Ausführungsform des chirurgischen Instruments ist das Anschlagselement durch eine Schraube gebildet. Dadurch wird beispielsweise der technische Vorteil erreicht, dass das Anschlagselement auf einfache Weise gebildet werden kann.

In einer weiteren technisch vorteilhaften Ausführungsform des chirurgischen Instruments ist die Spreizvorrichtung um die Schraube herum angeordnet. Dadurch wird beispielsweise ebenfalls der technische Vorteil erreicht, dass ein kompakter Aufbau erreicht wird.

In einer weiteren technisch vorteilhaften Ausführungsform des chirurgischen Instruments beträgt der maximale Spreizwinkel in der Reinigungsstellung 5°. Dadurch wird beispielsweise der technische Vorteil erreicht, dass eine gute Handhabbarkeit des chirurgischen Instruments in der Reinigungsstellung sichergestellt wird.

Gemäß der Erfindung ist das Schiebeteil in der Reinigungsstellung gegenüber dem Schaft unverschiebbar. Dadurch wird beispielsweise der technische Vorteil erreicht, dass ein unbeabsichtigtes Schließen des chirurgischen Instruments während der Reinigung verhindert werden kann.

In einer weiteren technisch vorteilhaften Ausführungsform des chirurgischen Instruments weist der Schaft ein Führungsprofil zum Führen des Schiebeteils auf. Dadurch wird beispielsweise der technische Vorteil erreicht, dass das Schiebeteil verliersicher an dem Schaft geführt ist.

In einer weiteren technisch vorteilhaften Ausführungsform des chirurgischen Instruments endet das Führungsprofil in einer Aussparung, in der das Schiebeteil freigegeben wird. Dadurch wird beispielsweise der technische Vorteil erreicht, dass dieses durch eine Aufklappbewegung durch das Spreizelement in die Reinigungsstellung bewegt werden kann.

In einer weiteren technisch vorteilhaften Ausführungsform des chirurgischen Instruments umfasst das chirurgische Instrument einen Druckknopf, der in der Arbeitsstellung einen Anschlag für ein Griffelement bildet. Dadurch wird beispielsweise der technische Vorteil erreicht, dass die Bewegung des Schiebeteils begrenzt wird.

In einer weiteren technisch vorteilhaften Ausführungsform des chirurgischen Instruments wird bei einem Betätigen des Druckknopfes der Anschlag freigegeben. Dadurch wird beispielsweise der technische Vorteil erreicht, dass das Schiebeteil in die Reinigungsstellung gelangen kann.

In einer weiteren technisch vorteilhaften Ausführungsform des chirurgischen Instruments ist der Druckknopf in dem Betätigungsgriff angeordnet. Dadurch wird beispielsweise der technische Vorteil erreicht, dass der Druckknopf auf einfache Weise betätigt werden kann.

In einer weiteren technisch vorteilhaften Ausführungsform des chirurgischen Instruments ist das chirurgische Instrument ein Laminektomie Rongeur. Dadurch wird beispielsweise der technische Vorteil erreicht, dass besonders geeignete chirurgische Werkzeuge verwendet werden.

Ausführungsbeispiele der Erfindung sind in den Zeichnungen dargestellt und werden im Folgenden näher beschrieben.

Es zeigen:
- Fig. 1: eine Darstellung eines chirurgischen Instruments in einer Arbeitsstellung;
- Fig. 2: eine Darstellung des chirurgischen Instruments in einer Reinigungsstellung; und
- Fig. 3: eine Darstellung des chirurgischen Instruments in der Arbeitsstellung.

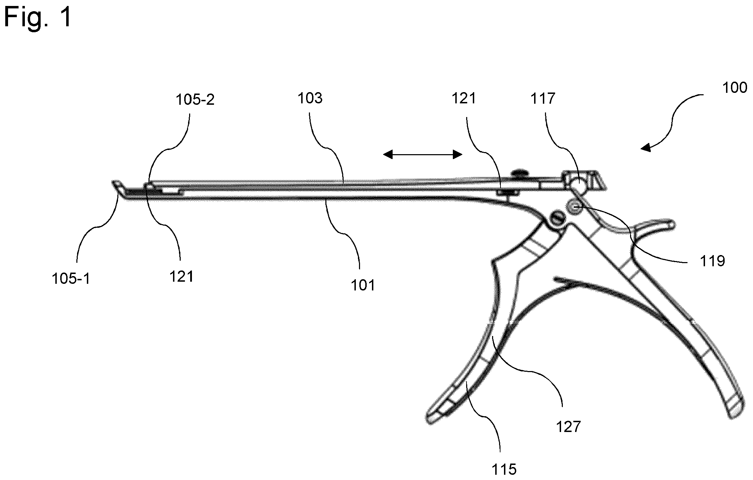

Fig. 1 eine Darstellung eines chirurgischen Instruments 100 in einer Arbeitsstellung. Das chirurgische Instrument 100 ist beispielsweise ein Laminektomie Rongeur und dient zum Abtragen von Knochengewebe an schwer zugänglichen Stellen. Hierzu umfasst das chirurgische Instrument 100 einen langgestreckten Schaft 101 mit einem ersten Schneidelement 105-1 und ein Schiebeteil 103 mit einem zweiten Schneidelement 105-2. Das Schiebeteil 103 ist auf dem Schaft 101 längsverschiebbar geführt.

Das chirurgische Instrument 100 umfasst einen Betätigungsgriff 115 mit einem drehbar gelagerten Griffelement 127. In einer Arbeitsstellung des chirurgischen Instruments 100 wird bei einem Betätigen des Betätigungsgriffes 115 das Schiebeteil 103 nach vorne geschoben, so dass sich die beiden Schneidelemente 105-1 und 105-2 aufeinander zu bewegen und dann schließen. Dadurch gelingt es, zwischen den beiden Schneidelementen 105-1 und 105-2 liegendes Knochengewebe zu entfernen. Wird der Betätigungsgriff 115 gelöst, bewegt sich das Schiebeteil 103 in die entgegengesetzte Richtung bis die Bewegung des Betätigungsgriffs 115 durch einen Druckknopf 119 begrenzt wird.

Die Schiebebewegung wird durch einen zylindrischen Kopf 117 von dem drehbar gelagerten Betätigungsgriff 115 auf das Schiebeteil 103 übertragen. Zu diesem Zweck ist das Schiebeteil 103 über ein nutenartiges Führungsprofil 121 an dem Schaft 101 verschiebbar geführt. In einem ausgesparten Endbereich 123 endet das Führungsprofil 121. In der Arbeitsstellung des chirurgischen Instruments 100 kann ein Nutenstein des Schiebeteils 103 die ausgesparten Endbereiche 123 nicht erreichen, so dass ein Lösen des Schiebeteils 103 von dem Schaft 101 ausgeschlossen ist.

Nach einer Verwendung des chirurgischen Instruments 100 wird dieses gereinigt und sterilisiert. Hierzu wird der Betätigungsgriff 115 leicht gedrückt, um eine Spannung von einem Druckknopf 119 zur Freigabe in die Reinigungsstellung zu nehmen. Durch die Betätigung des Druckknopfes 119 und das weitere Drücken des Betätigungsgriffes 115 wird das Schiebeteil 103 in eine Position außerhalb des Führungsprofils 121 an dem Schaft 101 zurückbewegt, bei der sich der Nutenstein in die ausgesparten Endbereiche 123 bewegt. Die Führung des Schiebeteiles 103 an dem Schaft wird dadurch gelöst.

Durch die manuelle Betätigung des Druckknopfes 119 und das gleichzeitige Drücken des vorgespannten Betätigungsgriffs 115 wird das chirurgische Instrument 100 entriegelt und gelangt dabei in eine Reinigungsstellung. Durch das anfängliche Drücken des vorgespannten Betätigungsgriffs 115 wird der Druckknopf 119 entlastet, so dass dieser ebenfalls manuell gedrückt und betätigt werden kann. Durch weiteres Betätigen des Betätigungsgriffs 115 bei gedrücktem Druckknopf 119 gelangt das Schiebeteil 103 anschließend in die Reinigungsstellung. Durch diesen Mechanismus kann sichergestellt werden, dass das chirurgische Instrument 100 nicht ungewollt von der Arbeitsstellung in die Reinigungsstellung gelangen kann.

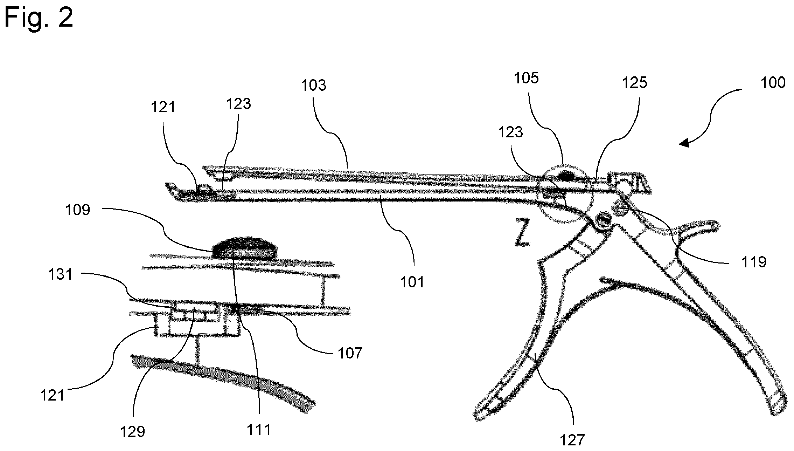

Fig. 2 zeigt eine Darstellung des chirurgischen Instruments 100 in der Reinigungsstellung. Durch die freigegebene Führung in der Reinigungsstellung öffnet sich das Schiebeteil 103 schnabelartig nach oben und klappt auseinander. Durch den auseinandergeklappten Schaft 101 und das Schiebeteil 103 ist es anschließend möglich, das chirurgische Instrument 100 im Bereich zwischen den beiden Elementen zu reinigen.

Das Auseinanderklappen wird durch eine Klappvorrichtung 105 erreicht, die eine Spreizvorrichtung 107 zum automatischen Spreizen des Schiebeteils 103 und des Schafts 101 umfasst. Die Spreizvorrichtung kann beispielsweise durch eine integrierte Spreizfeder 113 gebildet sein, die den Schaft 101 und das Schiebeteil 103 in der Reinigungsstellung automatisch auseinanderdrückt.

Die Spreizfeder 113 drückt das Schiebeteil 103 gegen ein Anschlagselement 109, das durch eine Schraube 111 mit einem Schraubenkopf gebildet ist. Die Schraube 111 ist an dem Schaft befestigt und befindet sich in einer länglichen Aussparung 125 innerhalb des Schiebeteils 103. Die Schraube 111 ist verdrehsicher befestigt, so dass diese ein fest positioniertes Anschlagselement 109 bildet und der maximale Öffnungswinkel in der Reinigungsstellung nicht verändert werden kann.

Durch bleibt das Schiebeteil 103 entlang des Schaftes 101 beweglich. Die Aussparung 125 ist größer als der Durchmesser der Schraube 111, so dass sich das Schiebeteil 103 in der Reinigungsstellung durch das Spiel der Schraube 111 in der Aussparung 125 auch seitlich bewegen kann.

Durch das Anschlagselement 109 wird der Aufklappwinkel zwischen dem Schaft 101 und dem Schiebeteil 103 begrenzt. Die Spreizfeder 113 ist zwischen dem Schaft 101 und dem Schiebeteil 103 und um die Schraube 111 herum angeordnet. Dies hat den Vorteil, dass die Spreizfeder 113 durch die Schraube 111 fixiert wird.

In der Reinigungsstellung kann das Schiebeteil 103 nicht verloren oder vertauscht werden. In der geöffneten Reinigungsstellung kann das Schiebeteil 103 auch bei Betätigung des Griffelementes 127 nicht nach vorne bewegt werden, sondern ist in der hinteren Stellung eingerastet. Auch eine seitliche Drehung des Schiebeteils 103 gegenüber dem Schaft 101 ist blockiert, so dass dieses lediglich in Richtung des Schaftes 101 zugeklappt werden kann.

Erst nach einem Zusammendrücken des Schiebeteils 103 und des Schaftes 101 lässt sich das Schiebeteil 103 bei einer Betätigung des Griffelementes 127 wieder entlang des Schaftes verschieben.

Dies wird dadurch erreicht, dass T-förmiger Nutenstein 129, der an dem Schiebeteil 103 angeordnet ist, in der Reinigungsstellung oberhalb des Führungsprofils 121 des Schaftes 101 liegt und beim Betätigen des Griffelementes 127 an eine Kante 131 anstößt. Dadurch wird eine Verschiebung des Schiebeteils 103 blockiert. Dies wird durch den fest vorgegebenen und unveränderbaren Öffnungswinkel in der Reinigungsstellung und die Spreizvorrichtung 107 erreicht. Erst nach dem Zusammendrücken des Schiebeteils 103 und des Schaftes 101 kann der Nutenstein 129 wieder von der internen Führung 121 aufgenommen werden, so dass das Schiebeteil 103 nach vorne bewegt werden kann.

Das chirurgische Instrument 100 ist einfach zerlegbar und handzuhaben und nach dem Reinigen einfacher zu montieren.

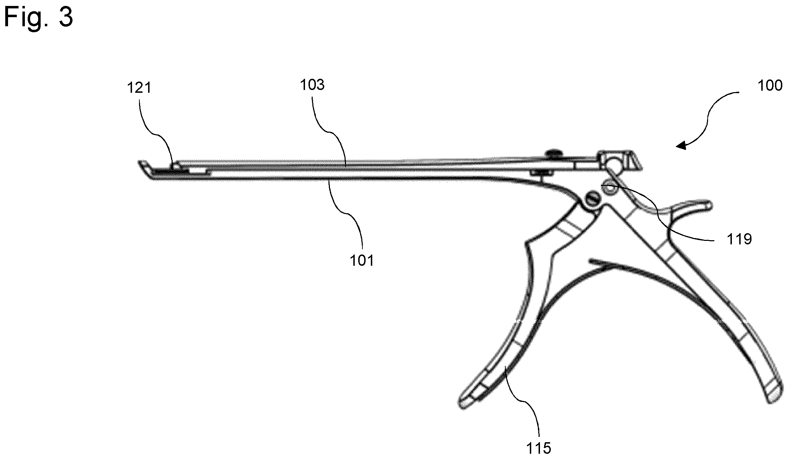

Fig. 3 zeigt eine erneute Darstellung des chirurgischen Instruments 100 in der Arbeitsstellung. Zum Erreichen der Arbeitsstellung wird das Schiebeteil 103 manuell nach unten gedrückt, so dass dieses wieder auf dem Schaft 101 zum Liegen kommt. Durch leichtes Betätigen des Betätigungsgriffes 115 bewegt sich das Schiebeteil 103 in Richtung des Führungsprofils 121, so dass die Führung des Schiebeteils wiederhergestellt wird. Das Schiebeteil 103 rastet erneut in der Arbeitsstellung ein. Auf diese Weise wird der technische Vorteil erreicht, dass eine Sicherung der Reinigungsstellung realisiert wird, bei der das Schiebeteil 103 nicht versehentlich verriegelt werden kann, wenn dieses nach oben geklappt ist.

Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Der Schutzbereich der vorliegenden Erfindung ist durch die Ansprüche gegeben und wird durch die in der Beschreibung erläuterten oder den Figuren gezeigten Merkmale nicht beschränkt.

### BEZUGSZEICHENLISTE

- 100: Chirurgisches Instrument
- 101: Schaft
- 103: Schiebeteil
- 105: Klappvorrichtung
- 107: Spreizvorrichtung
- 109: Anschlagselement
- 111: Schraube
- 113: Spreizfeder
- 115: Betätigungsgriff
- 117: Kopf
- 119: Druckknopf
- 121: Führungsprofil
- 123: Endbereich
- 125: Aussparung
- 127: Griffelement
- 129: Nutenstein

## Patentansprüche

1. Chirurgisches Instrument (100) zum Stanzen von Knochen, mit:
- einem Schaft (101) mit einem ersten Schneidelement (105-1) ;
- einem Schiebeteil (103) mit einem zweiten Schneidelement (105-2), das in einer Arbeitsstellung verschiebbar an dem Schaft (101) angeordnet ist; und
- einer Klappvorrichtung (105) zum schnabelartigen Aufklappen des Schiebeteils (103) und des Schafts (101) in einer Reinigungsstellung, die eine Spreizvorrichtung (107) zum Spreizen des Schiebeteils (103) und des Schafts (101) umfasst, wobei das Schiebeteil (103) in der Reinigungsstellung gegenüber dem Schaft (101) unverschiebbar ist und sich das Schiebeteil (103) erst nach einem Zusammendrücken des Schiebeteils (103) und des Schaftes (101) bei einer Betätigung eines Griffelementes (127) entlang des Schaftes (101) verschieben lässt,
**dadurch gekennzeichnet dass**
ein T-förmiger Nutenstein (129), der an dem Schiebeteil (103) angeordnet ist, in der Reinigungsstellung oberhalb eines Führungsprofils (121) des Schaftes (101) liegt und beim Betätigen des Griffelementes ( 127) an eine Kante (131) anstößt, so dass auch bei Betätigung des Griffelementes (127) das Schiebeteil (103) nicht nach vorne bewegt werden kann .

2. Chirurgisches Instrument (100) nach Anspruch 1, wobei die Spreizvorrichtung (107) eine Spreizfeder (113) umfasst.

3. Chirurgisches Instrument (100) nach einem der vorangehenden Ansprüche, wobei die Spreizvorrichtung (107) zwischen dem Schaft (101) und dem Schiebeteil (103) angeordnet ist.

4. Chirurgisches Instrument (100) nach einem der vorangehenden Ansprüche, wobei die Spreizvorrichtung (107) ausgebildet ist, das Schiebeteil (103) gegen einen Anschlagselement (109) zu drücken.

5. Chirurgisches Instrument (100) nach Anspruch 4, wobei das Anschlagselement (109) fest positioniert ist.

6. Chirurgisches Instrument (100) nach Anspruch 4 oder 5, wobei das Anschlagselement (109) durch eine Schraube (111) gebildet ist.

7. Chirurgisches Instrument (100) nach Anspruch 6, wobei die Spreizvorrichtung (107) um die Schraube (111) herum angeordnet ist.

8. Chirurgisches Instrument (100) nach einem der vorangehenden Ansprüche, wobei der maximale Spreizwinkel in der Reinigungsstellung 5° beträgt.

9. Chirurgisches Instrument (100) nach einem der vorangehenden Ansprüche, wobei der Schaft (101) ein Führungsprofil (121) zum Führen des Schiebeteils (103) aufweist.

10. Chirurgisches Instrument (100) nach Anspruch 9, wobei das Führungsprofil (121) in einer Aussparung endet, in der das Schiebeteil (103) freigegeben wird.

11. Chirurgisches Instrument (100) nach einem der vorangehenden Ansprüche, wobei das chirurgische Instrument (100) einen Druckknopf (119) umfasst, der in der Arbeitsstellung einen Anschlag für ein Griffelement (127) bildet.

12. Chirurgisches Instrument (100) nach Anspruch 11, wobei bei einem Betätigen des Druckknopfes (119) der Anschlag freigegeben wird.

13. Chirurgisches Instrument (100) nach Anspruch 11 oder 12, wobei der Druckknopf (119) in einem Betätigungsgriff (115) angeordnet ist.

14. Chirurgisches Instrument (100) nach einem der vorangehenden Ansprüche, wobei das chirurgische Instrument (100) ein Laminektomie Rongeur ist.

## Claims

1. A surgical instrument (100) for punching bones, comprising:
- a shaft (101) having a first cutting element (105-1);
- a sliding part (103) having a second cutting element (105-2), which is slidably arranged on the shaft (101) in a working position; and
- a folding device (105) for beak-like unfolding of the sliding part (103) and the shaft (101) in a cleaning position, which comprises a spreading device (107) for spreading the sliding part (103) and the shaft (101), wherein the sliding part (103) cannot be displaced relative to the shaft (101) in the cleaning position and the sliding part (103) can only be displaced after compression of the sliding part (103) and the shaft (101) upon actuation of a handle element (127) along the shaft (101),
**characterized in that**
a T-shaped slot nut (129), which is arranged on the sliding part (103), is located above a guide profile (121) of the shaft (101) in the cleaning position and abuts against an edge (131) when the handle element (127) is actuated so that even upon actuation of the handle element (127) the sliding part (103) cannot be moved towards the front.

2. The surgical instrument (100) according to claim 1, wherein the spreading device (107) comprises a spreading spring (113).

3. The surgical instrument (100) according to any one of the preceding claims, wherein the spreading device (107) is arranged between the shaft (101) and the sliding part (103).

4. The surgical instrument (100) according to any one of the preceding claims, wherein the spreading device (107) is configured to press the sliding part (103) against a stop element (109).

5. The surgical instrument (100) according to claim 4, wherein the stop element (109) is fixedly positioned.

6. The surgical instrument (100) according to claim 4 or 5, wherein the stop element (109) is formed by a screw (111).

7. The surgical instrument (100) according to claim 6, wherein the spreading device (107) is arranged around the screw (111).

8. The surgical instrument (100) according to any one of the preceding claims, wherein the maximum spread angle in the cleaning position is 5°.

9. The surgical instrument (100) according to any one of the preceding claims, wherein the shaft (101) has a guide profile (121) for guiding the sliding part (103).

10. The surgical instrument (100) according to claim 9, wherein the guide profile (121) terminates in a recess in which the sliding part (103) is released.

11. The surgical instrument (100) according to any one of the preceding claims, wherein the surgical instrument (100) comprises a push button (119) that forms a stop for a handle element (127) in the working position.

12. The surgical instrument (100) according to claim 11, wherein upon actuation of the push button (119) the stop is released.

13. The surgical instrument (100) according to claim 11 or 12, wherein the push button (119) is arranged in an actuation handle (115).

14. The surgical instrument (100) according to any one of the preceding claims, wherein the surgical instrument (100) is a laminectomy rongeur.

## Revendications

1. Instrument chirurgical (100) pour le poinçonnage des os, présentant :
- une tige (101) avec un premier élément de coupe (105-1) ;
- un élément coulissant (103) avec un deuxième élément de coupe (105-2), qui est disposé de manière coulissante dans une position de travail sur l'arbre (101) ; et
- un dispositif de rabattement (105) pour rabattre à la manière d'un bec l'élément coulissant (103) et la tige (101) dans une position de nettoyage, qui comprend un dispositif d'écartement (107) pour écarter l'élément coulissant (103) et la tige (101), où l'élément coulissant (103) est immobile en position de nettoyage par rapport à la tige (101) et l'élément coulissant (103) n'est déplacé le long de la tige (101) qu'après une compression ensemble de l'élément coulissant (103) et la tige (101) lors de l'actionnement d'un élément de préhension (127),
- **caractérisé en ce qu'**un coulisseau en forme de T (129), qui est disposé sur l'élément coulissant (103), se trouve en position de nettoyage au-dessus d'un profilé de guidage (121) de la tige (101) et lors de l'actionnement de l'élément de préhension (127) vient buter contre un bord (131), de manière que même lors de l'actionnement de l'élément de préhension (127), l'élément coulissant (103) ne peut pas être déplacé vers l'avant.

2. Instrument chirurgical (100) selon la revendication 1, où le dispositif d'écartement (107) comprend un ressort d'écartement (113).

3. Instrument chirurgical (100) selon l'une des revendications précédentes, où le dispositif d'écartement (107) est disposé entre la tige (101) et l'élément coulissant (103).

4. Instrument chirurgical (100) selon l'une des revendications précédentes, où le dispositif d'écartement (107) est conçu pour presser la partie coulissante (103) contre un élément de butée (109).

5. Instrument chirurgical (100) selon la revendication 4, où l'élément de butée (109) est positionné de manière fixe.

6. Instrument chirurgical (100) selon la revendication 4 ou 5, où l'élément de butée (109) est formé par une vis (111).

7. Instrument chirurgical (100) selon la revendication 6, où le dispositif d'écartement (107) est disposé autour de la vis (111).

8. Instrument chirurgical (100) selon l'une des revendications précédentes, où l'angle maximal d'écartement en position de nettoyage est de 5°.

9. Instrument chirurgical (100) selon l'une des revendications précédentes, où la tige (101) comporte un profilé de guidage (121) pour guider l'élément coulissant (103).

10. Instrument chirurgical (100) selon la revendication 9, où le profil de guidage (121) se termine par un évidement dans lequel l'élément coulissant (103) est libéré.

11. Instrument chirurgical (100) selon l'une des revendications précédentes, où l'instrument chirurgical (100) comprend un bouton-poussoir (119) qui forme une butée pour un élément de préhension (127) en position de travail.

12. Instrument chirurgical (100) selon la revendication 11, où lors d'un actionnement du bouton-poussoir (119) la butée est relâchée.

13. Instrument chirurgical (100) selon la revendication 11 ou 12, où le bouton-poussoir (119) est disposé dans une poignée d'actionnement (115).

14. Instrument chirurgical (100) selon l'une des revendications précédentes, où l'instrument chirurgical (100) est un rongeur de laminectomie.
